Europäisches Patentamt

(19) **European Patent Office**

**Office européen des brevets**

(11) Numéro de publication: **0 002 663**
**A2**

(12) # DEMANDE DE BREVET EUROPEEN

(21) Numéro de dépôt: 78101330.5

(51) Int. Cl.²: **G 02 C 7/04,** A 61 F 9/02

(22) Date de dépôt: **08.11.78**

(30) Priorité: **10.11.77 BE 182544**

(43) Date de publication de la demande: **11.07.79**
**Bulletin 79/14**

(84) Etats contractants désignés: **CH DE FR GB LU NL SE**

(71) Demandeur: **Bragard, Paul, Place du Parc 2, Liège (BE)**

(72) Inventeur: **Bragard, Paul, Place du Parc 2, Liège (BE)**

(74) Mandataire: **Degwert, Hartmut et al, c/o Bureau Gevers 7 rue de Livourne Bte 1, B-1050 Bruxelles (BE)**

(54) **Lentille de contact.**

(57)    La lentille de contact comporte un centre opaque qui s'oppose au passage des rayons lumineux. Elle permet une therapeutique nouvelle notamment de l'amblyopie par la neutralisation de l'œil valide au profit de l'œil affaibli.

EP 0 002 663 A2

ACTORUM AG

1

"LENTILLE DE CONTACT"

L'invention concerne une lentille de contact d'un type particulier permettant une therapeutique nouvelle et originale. Il est connu de corriger, notamment chez les enfants, certains défauts de la vue entraînant un affaiblissement de la vue d'un oeil, c'est le cas notamment de l'amblyopie par la neutralisation de l'oeil valide au profit de l'oeil affaibli.

Pour corriger cet état de choses il est courant de faire porter un cache ou un verre fumé au patient pour neutraliser l'oeil directeur. Pour diverses raisons d'ordre purement physique ou psychologique les enfants refusent le port soit de lunettes, soit d'un cache.

En particulier le port d'un cache qui constitue le seul écran réellement efficace, dans le cas de l'espèce est refusé par les enfants qui les arrachent pour des raisons d'ordre auxquelles il a été fait allusion. Quant aux verres mats ou semi-mats utilisés pour neutraliser un oeil ils sont d'une efficacité très relative et également peu esthétiques.

L'invention a pour but de remédier à ces inconvénients et de réaliser une pénalisation plus efficace d'un oeil dans tous les cas où la neutralisation de cet organe constitue un traitement correctif classique.

L'invention a donc pour objet de proposer une lentille de contact qui permet de remédier aux inconvénients des techniques utilisées jusqu'ici.

A cet effet la lentille de contact selon l'invention comporte un centre opaque s'opposant au passage des rayons lumineux.

Dans une forme de réalisation avantageuse le centre opaque précité a un diamètre légèrement supérieur à celui de la pupille de l'oeil.

D'autres détails et avantages de l'invention ressortiront de la description qui sera donnée ci-après d'une lentille de contact selon l'invention. Cette description n'est donnée qu'à titre d'exemple et ne limite pas l'invention.

La lentille de contact selon l'invention peut être une lentille souple ou rigide. Le centre opaque de la lentille destinée à s'opposer aux rayons lumineux peut être teintée dans la masse ou en surface. Le diamètre du centre opaque doit être légèrement supérieur au diamètre de la pupille. En pratique, le centre opaque de la lentille de contact aura un diamètre de 8 à 12 mm, afin que la pupille soit recouverte dans sa totalité.

Le centre opaque sera de couleur foncée de telle sorte que la prothèse ne se remarquera pratiquement pas. Ceci augmentera encore son intérêt puisqu'en dehors de sa grande efficacité thérapeutique elle offrira les très grands avantages d'ordre psychologique déjà cités dans le préambule.

En effet dans de nombreux cas le port de la prothèse passera inaperçu, ce qui est un but essentiel de l'objet de l'invention.

De la description qui précède on comprendra que la conjugaison des avantages d'ordre psychologique et thérapeutique est capitale et que la lentille de contact selon l'invention offre donc des possibilités infiniment plus intéressantes que les techniques de correction utilisées jusqu'à ce jour.

Il est évident que l'invention peut trouver application dans d'autres domaines que celui de la correction de l'amblyopie. En effet des lentilles à centre opaque selon l'invention pourraient être utilisées pour cacher certaines lésions ou défaut de la cornée par exemple.

REVENDICATIONS

1. Lentille de contact caractérisée en ce qu'elle comporte un centre opaque s'opposant au passage des rayons lumineux.

2. Lentille de contact selon la revendication 1 caractérisée en ce que le centre opaque précité à un diamètre légèrement supérieur à celui de la pupille de l'oeil.

3. Lentille de contact selon l'une quelconque des revendications 1 et 2 caractérisée en ce que le centre opaque précité est constitué par une zone teintée dans la masse.

4. Lentille de contact selon l'une quelconque des revendications 1 et 2 caractérisée en ce que le centre opaque précité est constitué par une zone teintée en surface.

5. Lentille de contact selon l'une quelconque des revendications 1 à 4 caractérisée en ce que le centre opaque est foncé.

6. Lentille de contact selon l'une quelconque des revendications 1 à 5 caractérisée en ce que le centre opaque a un diamètre de 8 à 12 mm.

7. Lentille de contact selon l'une quelconque des revendications 1 à 6 caractérisée en ce qu'elle est souple.

8. Lentille de contact selon l'une quelconque des revendications 1 à 6 caractérisée en ce qu'elle est dure.

9. Lentille de contact selon l'une quelconque des revendications 1 à 6 caractérisée en ce qu'elle est une combinaison d'une lentille dure et d'une lentille souple.

10. Lentille de contact selon l'une quelconque

- 2 -

des revendications 1 à 6 caractérisée en ce qu'elle présente un centre dur entouré d'une périphérie souple.